# EUROPÄISCHE PATENTSCHRIFT

(11) **EP 1 554 176 B1**
(45) Veröffentlichungstag und Bekanntmachung des Hinweises auf die Patenterteilung: **12.09.2007**
(21) Anmeldenummer: 03807746.7
(22) Anmeldetag: 10.07.2003
(51) Int. Cl.: B65B 3/02

(54) **FORMVERFAHREN, INSBESONDERE BLAS-UND/ODER VAKUUMFORMVERFAHREN, ZUM HERSTELLEN EINES MIT EINEM ABZUGEBENDEN MEDIUM GEFÜLLTEN ABGABEBEHÄLTERS**
MOULDING METHOD, IN PARTICULAR A BLOWING OR VACUUM MOULDING METHOD FOR PRODUCTION OF A DISPENSING CONTAINER FILLED WITH A MEDIUM FOR DISPENSING
PROCEDE DE MOULAGE, EN PARTICULIER PROCEDE DE MOULAGE PAR SOUFFLAGE ET/OU SOUS VIDE, POUR LA FABRICATION D'UN RECIPIENT DIFFUSEUR REMPLI D'UN MILIEU A DIFFUSER

(30) Priorität: 27.09.2002 DE 10245318
(43) Veröffentlichungstag der Anmeldung: 20.07.2005
(73) Patentinhaber: Hansen, Bernd, 74429 Sulzbach-Laufen (DE)
(72) Erfinder: Hansen, Bernd, 74429 Sulzbach-Laufen (DE)
(74) Vertreter: Bartels & Partner
(86) Internationale Anmeldenummer: PCT/EP2003/007452
(87) Internationale Veröffentlichungsnummer: WO 2004/033299

(56) Entgegenhaltungen:
- US-A- 2 667 165
- US-A- 2 667 872
- US-A- 5 538 506
- US-A- 5 687 550

## Beschreibung

Die Erfindung bezieht sich auf ein Formverfahren, insbesondere Blas- und/oder Vakuumformverfahren, zum Herstellen eines mit einem abzugebenden Medium gefüllten Abgabebehälters. Insbesondere richtet sich die Erfindung auf die Herstellung solcher Abgabebehälter, deren hauptsächlicher, jedoch nicht ausschließlicher Verwendungszweck darin besteht, ein gewünschtes Volumen des Mediums, insbesondere eines flüssigen Mediums, in einen Aufnahmebehälter einzubringen. Vorzugsweise handelt es sich bei dem einzubringenden Medium um einen Zusatzstoff, der beispielsweise als Wirkstoffbeimengung in ein im Aufnahmebehälter befindliches Fluid eingebracht wird. Bei dem Aufnahmebehälter kann es sich um einen Infusionsbehälter handeln, zu dessen Inhalt das Medium als zusätzlicher Wirkstoff beizumengen ist.

Als Abgabebehälter wird hierzu üblicherweise eine Spritze benutzt, mit deren Kanüle ein perforierbarer Verschluß oder Stopfen des Aufnahmebehälters, beispielsweise des Infusionsbehälters, durchstochen wird, wonach durch Ausdrücken der Spritze das Medium in den Aufnahmebehälter injiziert wird. Bei diesem Vorgehen ist der vorbereitende Arbeitsschritt des Befüllens der Spritze erforderlich, indem die gewünschte Menge des Mediums aus einem Vorratsbehälter in die Spritze umgefüllt oder die Spritze aus einer konventionellen Phiole, die die abgemessene Dosis des betreffenden Mediums enthält, befüllt wird. Diese Arbeitsschritte des Umfüllens sind zum einen zeitraubend, weil Kanüle und Spritze ausgepackt, die Kanüle auf die Spritze montiert, die Phiole geöffnet oder angestochen werden und die Spritze aufgezogen werden müssen. Zum anderen besteht bei diesen Maßnahmen ein erhebliches Risiko der Kontamination des Mediums.

Verfahren, die die simultane Ausbildung und Befüllung von Behältern durch Blasformen oder Vakuumformen ermöglichen, sind bereits bekannt. So zeigen beispielsweise die DE 197 07 292 A1 und die US 5687550 ein derartiges Blasformverfahren. Die Erfindung stellt sich die Aufgabe, auf Grundlage eines derartigen Blasformverfahrens oder eines entsprechenden Vakuumformverfahrens ein Herstellungsverfahren für Abgabebehälter aufzuzeigen, das nicht nur eine besonders einfache und rationelle Herstellung der Behälter ermöglicht, sondern insbesondere auch für die Herstellung solcher Abgabebehälter vorgesehen ist, die eine besonders einfache, schnelle und sichere Abgabe des Mediums aus dem Abgabebehälter in einen Aufnahmebehälter ermöglichen.

Diese Aufgabe ist erfindungsgemäß durch ein Formverfahren gelöst, das die Merkmale des Anspruches 1 aufweist.

Dadurch, dass erfindungsgemäß sowohl die Ausbildung des Behälters und dessen Befüllen mit dem betreffenden Medium als auch das Verschließen des gefüllten Behälters, einschließlich des Einlegens einer für den Abgabevorgang speziell ausgebildeten Verschlußeinheit mit einer aus mehreren Bestandteilen bestehenden Sicherungseinrichtung, innerhalb einer Formeinrichtung erfolgt, also an ein und demselben Herstellungsort, aus dem erst der vollständig gefertigte Abgabebehälter wegtransportiert zu werden braucht, ergibt sich eine besonders einfache Herstellung, bei der die zu fordernde Sterilität ohne Schwierigkeiten zu gewährleisten ist.

Dadurch, dass bei dem mit dem erfindungsgemäßen Verfahren hergestellten, das Medium enthaltenden Abgabebehälter die integrierte Kanüle sowie eine deren vorstehendes Ende, also die Nadelspitze, abdeckende Sicherungseinrichtung zu einer Gesamteinheit zusammengefaßt sind, kann die gewünschte Dosis des betreffenden Mediums vor dem Abgabevorgang im Abgabebehälter bereitgestellt werden. Da die Nadelspitze durch die Sicherungseinrichtung abgedeckt ist, kann der Behälter mit gesicherter Kanüle ohne weiteres in seinem für den Abgabevorgang bereiten Zustand gehandhabt werden. Zwischenschritte des Umfüllens von Medium und des Bereitmachens einer Spritze sind für den Abgabevorgang nicht erforderlich. Die erstrebte Vereinfachung, Zeitersparnis und erhöhte Sicherheit gegen Kontamination sind somit erreicht. Bei nach dem erfindungsgemäßen Verfahren hergestellten Behältern ist nicht nur die Gefahr der Kontamination des Mediums stark reduziert, sondern auch die Gefahr verringert, dass sich der Benutzer, etwa eine Krankenschwester, verletzt, weil die Kanüle nach Gebrauch wieder durch die Sicherungseinrichtung geschützt ist.

Wenn das äußere Ende der Kanüle dazu vorgesehen ist, einen perforierbaren Verschluß eines Aufnahmebehälters zu durchstoßen, der das abzugebende Medium aufnehmen soll, kann die Sicherungseinrichtung der Verschlußeinheit so gestaltet sein, dass nach dem Abnehmen der Schutzhaube der Kanülen-Schutzkörper beim Durchstoßen des Verschlusses durch Anlage an demselben aus seiner vorgeschobenen Schutzstellung am Ende der Kanüle in die Gebrauchsstellung zurückschiebbar und nach Herausziehen der Kanüle aus dem Verschluß wieder in die Schutzstellung vorschiebbar ist. Dadurch ist die Handhabung beim Abgabevorgang besonders vereinfacht.

Bei einem besonders vorteilhaften Ausführungsbeispiel des Formverfahrens werden die Schritte des Aufweitens und des Füllens des in der Form befindlichen Behälters gemeinsam mittels eines die Einführöffnung durchgreifenden, kombinierten Blas-Fülldornes ausgeführt. Dadurch wird eine besonders rationelle Herstellung der gefüllten Behälter mit kurzen Taktzeiten ermöglicht.

Im Zuge des Formens der Schutzhaube als integrierten Teil des Behälters und als Bestandteil der Schutzeinrichtung wird vorzugsweise beim Schließen der Kopfbacken der Form im Bereich des Überganges zwischen Verschlußeinheit und Schutzhaube an deren Wand eine Sollbruchstelle ausgeformt, die eine das Abnehmen der Schutzhaube erleichternde Trennstelle bildet.

Vorzugsweise wird beim Schließen der Kopfbacke an der Schutzhaube zumindest ein vorspringendes Griffstück ausgeformt, das einen Abdrehknebel für bequemes manuelles Abtrennen der Schutzhaube bildet.

Nachstehend ist die Erfindung anhand der Zeichnung im einzelnen erläutert. Es zeigen:
- Fig. 1 bis 3 stark schematisch vereinfacht gezeichnete Darstellungen wesentlicher Teile einer Formeinrichtung zum Durchführen des erfindungsgemäßen Verfahrens, wobei in diesen Fig. mehrere aufeinander folgende Schritte des Verfahrensablaufs verdeutlicht sind;
- Fig. 4 einen vergrößert gezeichneten Ausschnitt zur Verdeutlichung des Verfahrensschrittes des Einlegens einer dem Behälter zugehörigen Verschlußeinheit;
- Fig. 5 eine im Maßstab den Fig. 1 bis 3 entsprechende Darstellung der Formeinrichtung, wobei der Schritt des Formens einer die Verschlußeinheit umgebenden Schutzhaube verdeutlicht ist;
- Fig. 6 eine Seitenansicht eines Ausführungsbeispiels eines mit dem erfindungsgemäßen Formverfahren hergestellten Abgabebehälters in dem Betriebszustand vor dem Gebrauch, d. h. mit aufgesetzter Schutzhaube;
- Fig. 7 einen Längsschnitt des Abgabebehälters von Fig. 6;
- Fig. 8 einen der Fig. 7 entsprechenden Längsschnitt, jedoch mit abgenommener Schutzhaube;
- Fig. 9 einen Längsschnitt des Abgabebehälters in einem Betriebszustand, bei dem eine Abgabekanüle des Abgabebehälters in einen perforierbaren Verschlußstopfen eines Infusionsbehälters eingestochen ist;
- Fig. 10 eine der Fig. 9 ähnliche Darstellung, wobei jedoch der Abgabebehälter zur Abgabe enthaltenen Mediums ausgedrückt ist;
- Fig. 11 einen Längsschnitt des Abgabebehälters in ausgedrücktem und aus dem Verschlußstopfen abgezogenem Zustand und
- Fig. 12 einen Längsschnitt des Abgabebehälters im ausgedrückten, der Schutzstellung eines der Bestandteile der Sicherungseinrichtung entsprechenden Betriebszustand.

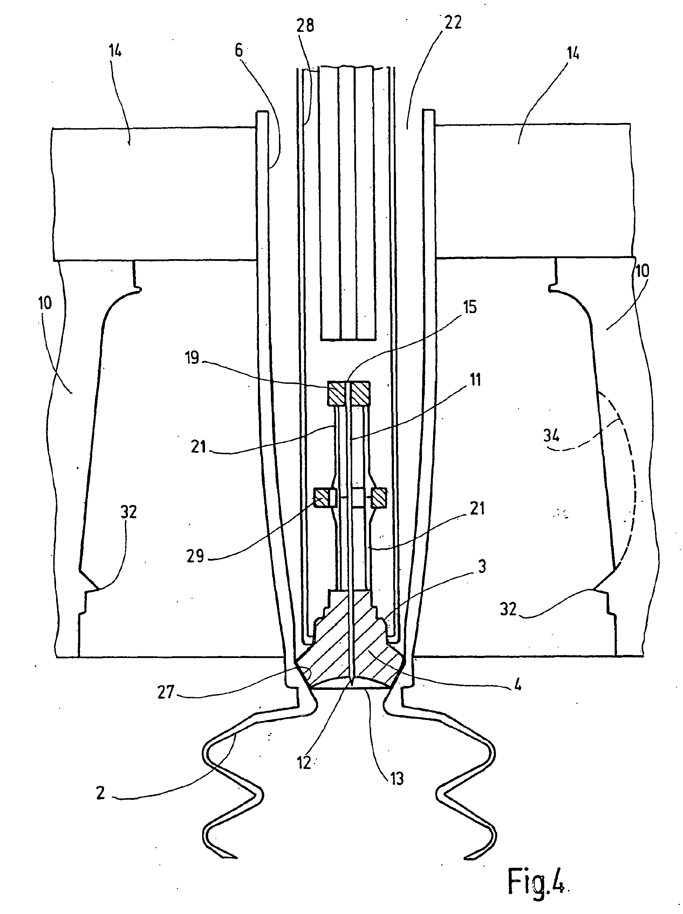

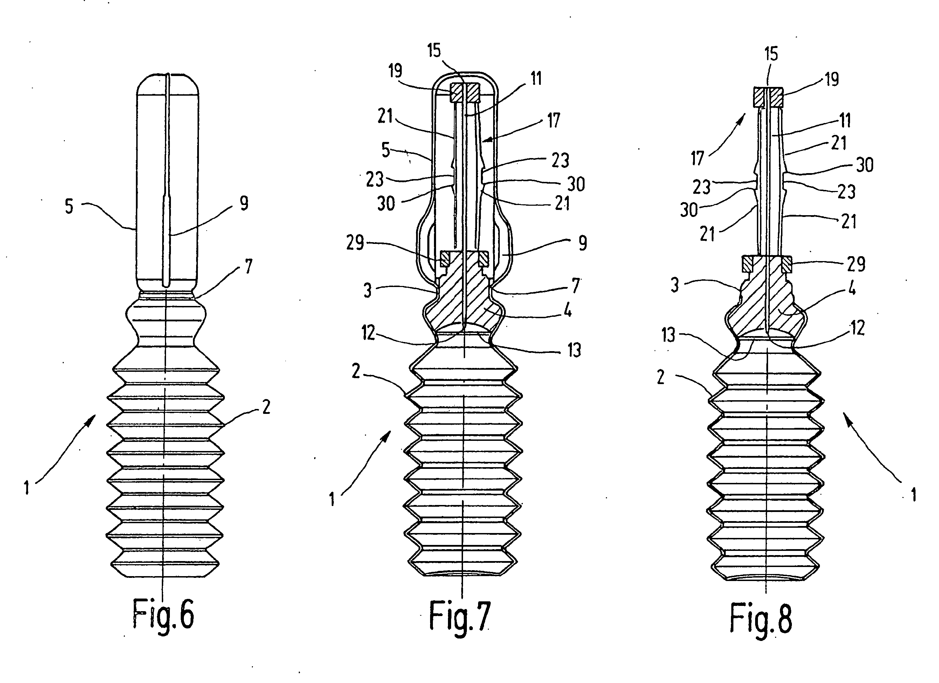

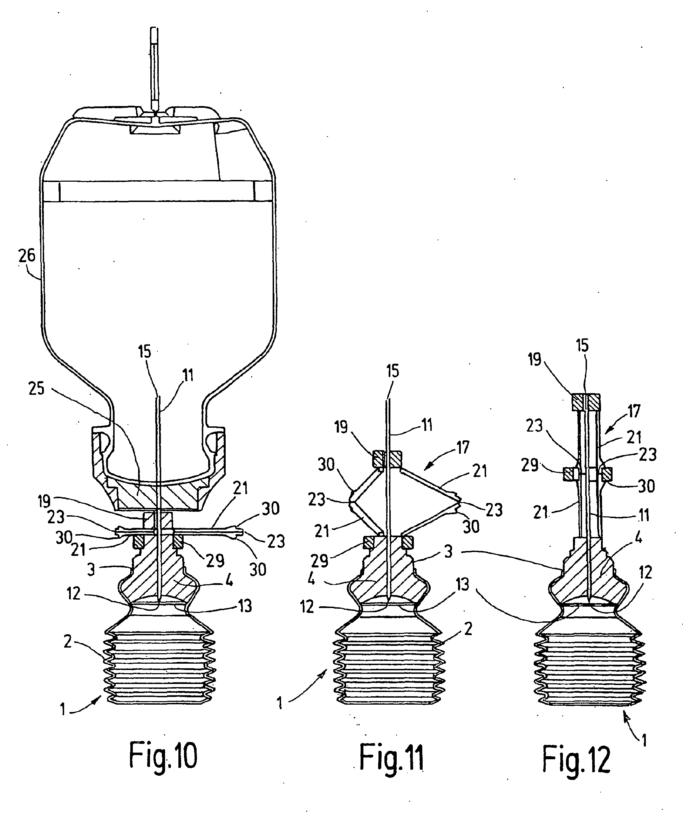

In den Figuren ist ein Ausführungsbeispiel eines mit dem erfindungsgemäßen Verfahren hergestellten, gefüllten Abgabebehälters als Ganzes mit 1 bezeichnet. Bei dem gezeigten Ausführungsbeispiel handelt es sich um einen ampullenartigen Kunststoffbehälter, dessen Wand 2 mit Falten versehen und balgartig gestaltet ist, so dass der Abgabebehälter 1 aus der in Fig. 6 bis 9 gezeigten Konfiguration zusammengedrückt werden kann, siehe Fig. 10 bis 12. Im Halsbereich ist in den Abgabebehälter 1 als Einlegeteil eine Verschlußeinheit 3 eingesetzt. Wie am deutlichsten aus Fig. 4, 7 und 8 zu ersehen ist, ist der Körper 4 der Verschlußeinheit 3 im Zentralbereich von einer Kanüle 11 durchzogen, deren inneres Ende 12 über den Körper 4 der Verschlußeinheit 3 nach innen leicht vorsteht. Zwischen dem inneren Ende 12 der Kanüle 11 und dem Innenraum des Abgabebehälters 1 befindet sich eine Membran 13, die Teil des Einlegeteils der Verschlußeinheit 3 ist. Am Körper 4 der Verschlußeinheit 3 ist außerdem ein sich entlang des vorstehenden Teiles der Kanüle erstreckender erster Bestandteil 17 einer Kanülen-Sicherungseinrichtung gelagert. Zweiter Bestandteil dieser Sicherungseinrichtung ist eine den vorstehenden Teil der Kanüle 11 umhüllende Schutzhaube 5, die bei der Herstellung des Behälters 1 als integraler Bestandteil desselben gebildet wird. Nachstehend ist die Erfindung am Beispiel eines Blasformverfahrens erläutert. Die Formung des Behälters mit Schutzhaube 5 könnte auch durch ein Vakuumformverfahren oder ein kombiniertes Blas-/Vakuumformverfahren erfolgen.

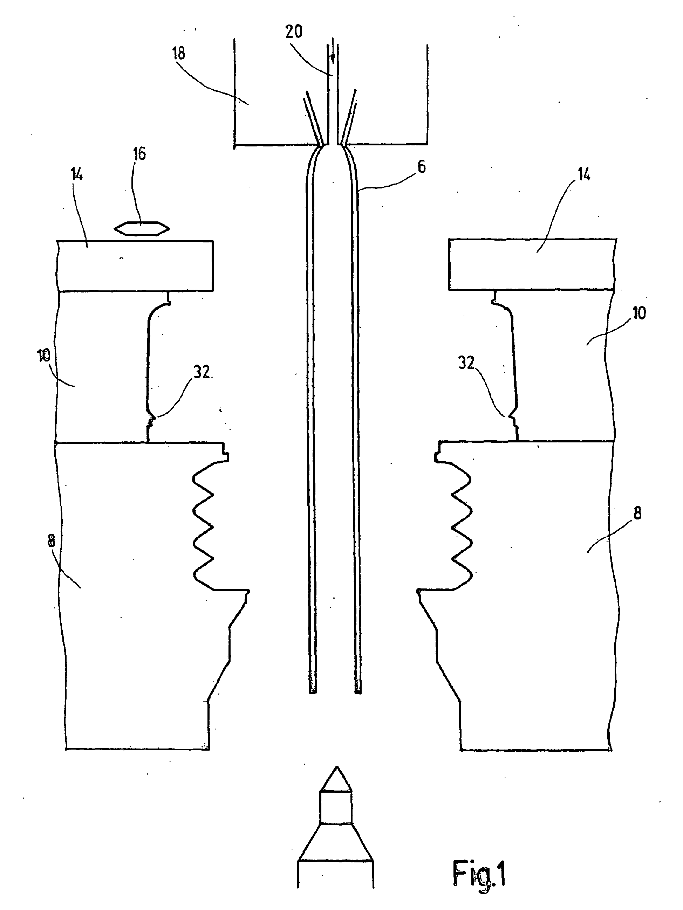

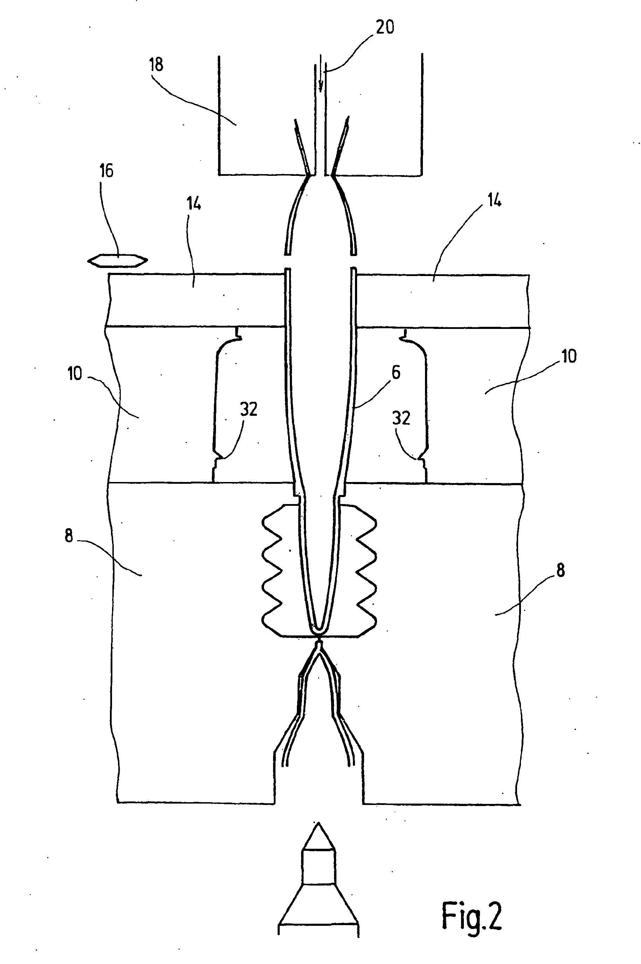

Der Ablauf des Formverfahrens ist in Fig. 1 bis 5 verdeutlicht, in denen die wesentlichen Teile einer Formeinrichtung stark schematisch vereinfacht dargestellt sind. Die Einrichtung weist drei Paare beweglicher Formbacken auf, nämlich Hauptformbacken 8 zur Formung des ein abzugebendes Medium aufnehmenden Behälter-Hauptteiles, Kopfbacken 10 zur Bildung des die Verschlußeinheit 3 umschließenden oberen Behälterteiles, beim vorliegenden Beispiel der Schutzhaube 5 der Sicherungseinrichtung, sowie Haltebacken 14 zur Stabilisierung eines extrudierten Kunststoffschlauches 6. Dieser wird aus einer Düse 18, die in an sich bekannter Weise einen Anschluß 20 für die Zufuhr von Stützluft aufweist, in die in Fig. 1 vollständig geöffnete Form hinein extrudiert, wonach die Hauptformbacken 8 geschlossen und die Haltebacken 14 an den Schlauch 6 heran bewegt werden, die den Schlauch 6 mittels Vakuum in stabiler Form halten, der im Abschnitt zwischen Düse 18 und Haltebacken 14 mittels des Messers 16 durchtrennt wird. Das so erreichte Stadium des Verfahrensablaufs ist in Fig. 2 gezeigt, aus der auch erkennbar ist, dass der Schlauch 6 durch das Schließen der Hauptformbacken 8 am voreilenden Endbereich zur Bildung des geschlossenen Behälterbodens verschweißt wird.

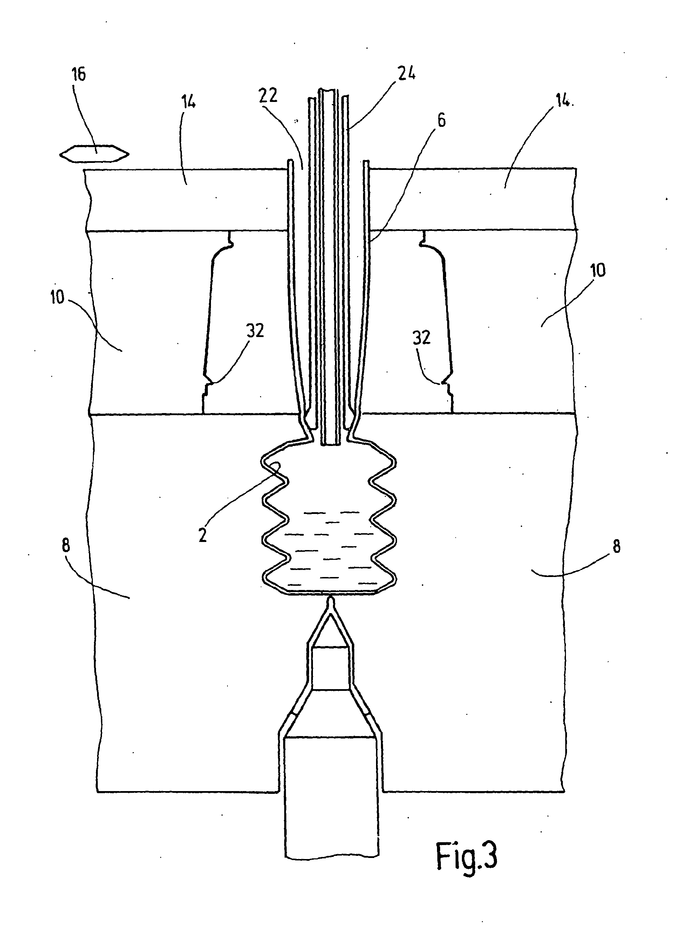

Fig. 3 zeigt, dass durch die Einführöffnung 22 hindurch, die durch das Durchtrennen des Schlauches 6 gebildet ist, ein beweglicher, kombinierter Blas-Fülldorn 24 eingefahren ist, durch den das Aufweiten des Schlauches 6 mittels Blasluft erfolgt, so dass sich die Behälterwand 2 in balgartiger Konfiguration an die Wände der Hauptformbacken 8 anschmiegt, und dass sodann, nachdem so der Innenraum des Behälters geformt ist, das abzugebende Medium mittels des kombinierten Blas-Fülldornes 24 eingefüllt wird, siehe Fig. 3.

Als nächster Verfahrensschritt schließt sich das Einlegen der Verschlußeinheit 3 mittels eines durch die Einführöffnung 22 einfahrbaren, beweglichen Sauggreifers 28 an, siehe Fig. 4. Wie aus dieser Fig. ersichtlich, weist der Körper 4 der Verschlußeinheit 3 eine Kegelfläche auf, die an einem Sitz 27 anliegt, den die Innenwand des Schlauches 6 in dem Bereich bildet, in dem die Formwände der Hauptformbacken 8 an die Kopfbacken 10 angrenzen.

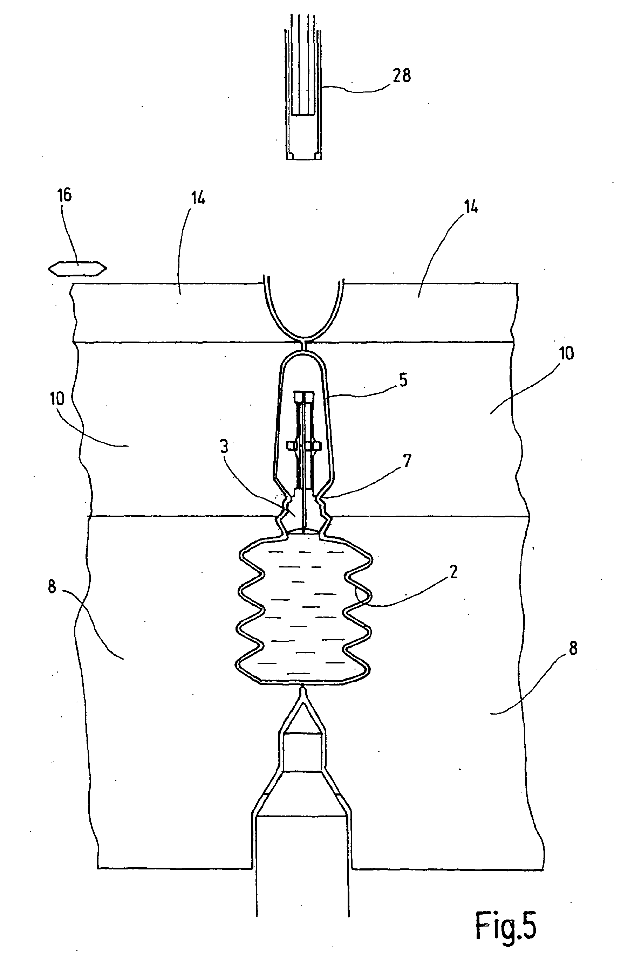

Fig. 5 zeigt, dass im weiteren. Verlauf der Sauggreifer 28 nach erfolgtem Einlegevorgang nach oben weggefahren ist und die Form durch zusammenfahren der Kopfbacken 10 nunmehr geschlossen ist, wodurch der sich im Bereich der Kopfbacken 10 erstreckende Abschnitt des Schlauches 6 zur Schutzhaube 5 geformt ist.

Wie am deutlichsten aus der in vergrößertem Maßstab gezeichneten Teildarstellung von Fig. 4 entnehmbar ist, weisen die Kopfbacken 10 einen Formvorsprung 32 auf, der beim Schließen der Kopfbacken 10 am Schlauch 6 eine ringförmige Kerbe ausformt, die eine Sollbruchstelle 7 bildet, an der die Schutzhaube 5 bequem vom restlichen Behälter zu trennen ist. Wie aus Fig. 6 und 7 ersichtlich ist, ist an der Außenseite der Schutzhaube 5 ein Drehknebel 9 als Handhabe ausgeformt, die ein bequemes manuelles Abdrehen der Schutzhaube 5 ermöglicht. Die Kopfbacken 10 weisen an diametral gegenüberliegenden Stellen Vertiefungen auf, um zwei einander gegenüberliegende Drehknebel 9 auszuformen. Von diesen Vertiefungen ist lediglich in Fig. 4 eine Vertiefung 34 mit gestrichelter Linie angedeutet.

Nähere Einzelheiten der Kanülen-Sicherungseinrichtung, die, wie erwähnt, die Schutzhaube 5 als einen Bestandteil und den in Fig. 4 sowie 7 bis 12 näher gezeigten, weiteren Bestandteil 17 aufweist, werden nun unter Bezug auf Fig. 7 bis 12 näher erläutert.

Die Kanüle 11 erstreckt sich vom äußeren Ende des Körpers 4 der Verschlußeinheit 3 in einer Länge, die in etwa der Länge einer Spritzenkanüle entspricht. Die Fig. 6 und 7 zeigen Betriebszustände, bei denen das vorstehende äußere Ende 15 der Kanüle 11 jeweils durch beide Bestandteile der Sicherungseinrichtung abgedeckt ist also sowohl durch die Schutzhaube 5 als auch durch den als Ganzes mit 17 bezeichneten Bestandteil.

Dieser Bestandteil 17 ist an den Körper 4 der Verschlußeinheit 3 einstückig angeformt und weist einen Ringkörper 19 auf, der auf der Kanüle 11 verschiebbar ist und sich in der Schutzstellung, siehe Fig. 7, 8 und 12, am äußeren Ende 15 der Kanüle 11 befindet, um dieses Kanülenende, d.h. die Nadelspitze, abzudecken. Der Ringkörper 19 ist über einstückig angeformte Trägerelemente 21 stabartiger Gestalt mit dem Körper 4 der Verschlußeinheit 3 einstückig verbunden, wobei die Verbindungsstellen mit Ringkörper 19 und Körper 4 der Verschlußeinheit 3 in der Art von Biegegelenken nachgiebig sind. Außerdem befinden sich auf etwa der halben Länge der Trägerelemente 21 Biegegelenke 23, die die Trägerelemente 21 unterteilen. Wird der Ringkörper 19 aus der Schutzstellung in die Gebrauchsstellung des Abgabebehälters längs der Kanüle 11 verschoben, siehe Fig. 10 und 11, so schwenken sich die an die Biegegelenke 23 angrenzenden Abschnitte der Trägerelemente 21, so dass sie sich von der Kanüle 11 abspreizen und so zusammenlegen, wie dies in Fig. 10 gezeigt ist.

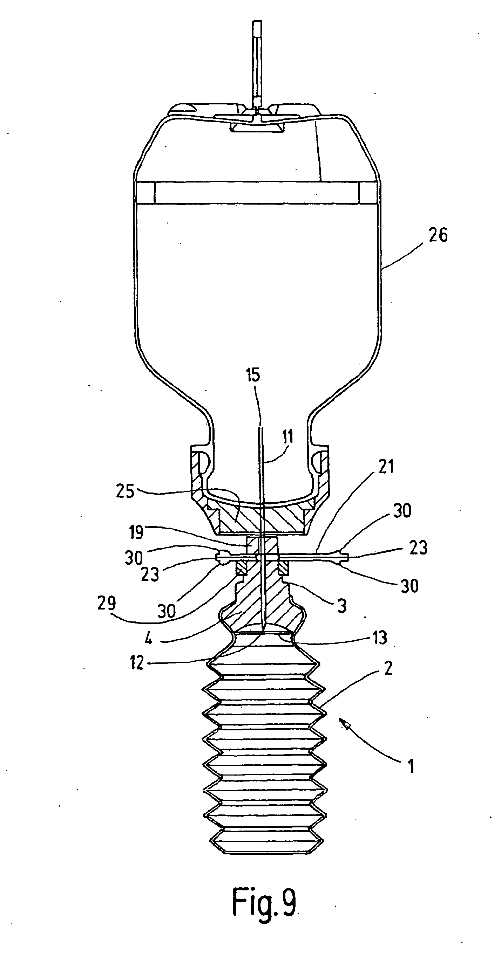

Die letztgenannten Fig. 10 und Fig. 9 zeigen den Behälter in dem Zustand, wo die Kanüle 11 mit ihrem vorderen Ende 15 einen perforierbaren Verschluß 25 eines Infusionsbehälters 26 durchstochen hat. Hierbei ist der Ringkörper 19 aus der auf das Ende 15 der Kanüle ausgerichteten Schutzstellung längs der Kanüle 11 in die Gebrauchsstellung zurückgeschoben. Durch Zusammendrücken der balgartigen Wand 2 des Abgabebehälters 1 (siehe Fig. 10) ist der Druck des darin befindlichen Mediums erhöht worden, so dass die Membran 13 gegen das zugewandte Ende 12 der Kanüle 11 gepreßt und von dieser durchstochen wird. Das Zusammendrücken des Abgabebehälters 1 führt dadurch zu einem Ausdrücken des darin befindlichen Mediums in den Infusionsbehälter 26 hinein, so dass zu dessen Inhalt eine dem Inhalt des Abgabebehälters 1 entsprechende Dosis eines Zusatz-oder Wirkstoffes beigemengt wird. Für das Durchstoßen der Membran 13 könnte die Kanüle 11 im Körper 4 der Verschlußeinheit 3 auch für eine durch Anschläge (nicht gezeigt) begrenzte Verschiebebewegung so geführt sein, dass die Kanüle 11 beim Durchstoßen des Verschlusses 25 so weit zurückgeschoben wird, dass ihr Ende 12 die Membran 13 perforiert.

Fig. 11 zeigt den Betriebszustand, nachdem der ausgedrückte Abgabebehälter 1 wieder aus dem Verschluß 25 des Infusionsbehälters 26 abgezogen ist. Aufgrund der Eigenelastizität der Trägerelemente 21 hat sich der Ringkörper 19, welcher zuvor beim Einstechen der Kanüle in den Verschluß 25 durch Anlage an denselben gegen die Elastizitäts- oder Haltekraft der Trägerelemente 21 aus der Schutzstellung zurückgeschoben worden ist, selbsttätig durch die Elastizitätskraft wieder teilweise gegen das Ende 15 der Kanüle 11 hin vorgeschoben.

Fig. 12 zeigt den Betriebszustand des Behälters nach erfolgtem Gebrauch desselben, wobei das vorstehende äußere Ende 15 de Kanüle 11, obgleich die Schutzhaube 5 nicht mehr angebracht ist, durch den Bestandteil 17 der Sicherungseinrichtung wieder gesichert ist. Zu diesem Zweck ist ein auf dem Körper 4 der Verschlußeinheit 3 abnehmbar sitzender Sicherungsring 29 von dem Körper 4 der Verschlußeinheit abgenommen und längs der Kanüle 11 vorgeschoben, wobei er über die Trägerelemente 21 gleitet und diese aus der in Fig. 11 gezeigten Position an die Kanüle 11 annähert, wobei der Ringkörper 19 bis zum Ende 15 der Kanüle 11 vorgeschoben wird. An den Biegegelenken 23 weisen die Trägerelemente 21 angeformte Rastkerben 30 auf, in die der Sicherungsring 29 einrastet, siehe Fig. 12.

Nach dem Einrasten des Sicherungsringes 29 in die Rastkerben 30 an den Biegegelenken 23 der Trägerelemente 21 ist die Kanüle 11 durch den ihr Ende 15 abdeckenden Ringkörper 19 trotz abgenommener Schutzhaube 5 wiederum abgedeckt, so dass der nun entleerte Behälter gefahrlos entsorgt werden kann. Es versteht sich, dass der Abgabebehälter nicht nur zur Beimengung gewünschter Volumina flüssiger Medien in Infusionsbehälter mit Vorteil benutzt werden kann, sondern gleichermaßen für die Abgabe flüssiger, halbfester oder gasförmiger und/oder partikelbefrachteter Medien, soweit deren Abgabe über Kanülen möglich oder erforderlich ist, angewendet werden kann.

## Patentansprüche

1. Formverfahren, insbesondere Blas- und/oder Vakuumformverfahren, zum Herstellen eines mit einem abzugebenden Medium gefüllten Abgabebehälters (1), welches die Schritte aufweist:
- Einbringen eines extrudierten Kunststoffschlauches (6)in eine geöffnete Form, die bewegliche Hauptformbacken (8) und Kopfbacken (10) aufweist,
- Durchtrennen des Kunststoffschlauches (6) im außerhalb der Kopfbacken (10) befindlichen Bereich zur Bildung einer Einführöffnung (22),
- Schließen der Hauptformbacken (8) zur Bildung des Formhohlraumes für den Abgabebehälter (1) und dabei erfolgendes Verschweißen des Kunststoffschlauches (6) an dessen vorderem Bereich zur Bildung des Behälterbodens,
- Aufweiten des Kunststoffschlauches (6) zur Anlage an den Formwänden der Hauptformbacken (8) durch Einblasen von Luft durch die Einführöffnung (22) und/oder durch Erzeugen eines Vakuums an den Formwänden,
- Füllen des in der Form befindlichen Behälters (1) mit dem abzugebenden Medium durch die Einführöffnung (22),
- Einlegen einer solchen Verschlußeinheit (3) durch den sich durch die Kopfbacken (10) erstreckenden Abschnitt des Kunststoffschlauches (6) hindurch, welche eine aus ihrem äußerem Ende vorstehende Abgabekanüle (11) für das Medium besitzt, die eine Sicherungseinrichtung (5,17) aufweist, deren erster Bestandteil (17) einen Kanülen-Schutzkörper (19) besitzt, der auf der.Kanüle (11) zwischen einer vorgeschobenen Schutzstellung und einer zurückgezogenen Gebrauchsstellung verschiebbar ist, und
- Schließen der Kopfbacken (10) und **dadurch** erfolgendes Formen des sich durch die Kopfbacken (10) erstreckenden Abschnittes des Kunststoffschlauches (6) zu einer Schutzhaube (5), die die Kanüle (11) der Verschlußeinheit (3) als zweiter Bestandteil der Sicherungseinrichtung (5,17) umschließt.

2. Formverfahren nach Anspruch 1, **dadurch gekennzeichnet, dass** die Schritte des Aufweitens und des Füllens des in der Form befindlichen Behälters (1) gemeinsam mittels eines die Einführöffnung (22) durchgreifenden, kombinierten Blas-Fülldornes (24) ausgeführt werden.

3. Formverfahren nach Anspruch 1 oder 2, **dadurch gekennzeichnet, dass** beim Schließen der Kopfbacken (10) der Form im Bereich des Überganges zwischen Verschlußeinheit (3) und Schutzhaube (5) an deren Wand eine Sollbruchstelle (7) ausgeformt wird.

4. Formverfahren nach Anspruch 3, **dadurch gekennzeichnet, dass** beim Schließen der Kopfbacken (10) an der Schutzhaube (5) zumindest ein vorspringendes Griffstück (9) als Abdrehknebel zum Abtrennen der Schutzhaube (5) an der Sollbruchstelle (7) ausgeformt wird.

5. Formverfahren nach Anspruch 3 oder 4, **dadurch gekennzeichnet, dass** die Verschlußeinheit (3) mit ihrem Verschlußkörper (4) in einen Sitz (27) eingelegt wird, der durch die den Kopfbacken (10) benachbarten Formwandabschnitte der geschlossenen Hauptformbacken (8) gebildet wird.

6. Formverfahren nach Anspruch 5, **dadurch gekennzeichnet, dass** eine verschlußeinheit (3) eingelegt wird, deren Verschlußkörper (4) sich von dem durch die Hauptformbacken (8) gebildeten Sitz (27) längs der Kanüle (11) in den Bereich der.Kopfbacken (10) erstreckt.

7. Formverfahren nach Anspruch 6, **dadurch gekennzeichnet, dass** die Sollbruchstelle (7) in einem Abstand vom Sitz (27) des Verschlußkörpers (4) so ausgebildet wird, dass sie den Verschlußkörper (4) in dem in die Kopfbacken (10) ragenden Bereich umringt.

## Claims

1. Moulding method, in particular blow and/or vacuum moulding method, for producing a dispensing container (1) filled with a medium that is to be dispensed, comprising the following steps:
- insertion of an extruded plastic hose (6) into an open mould comprising moveable main mould jowls (8) and header jowls (10),
- cutting the plastic hose (6) in the area outside of the header jowls (10) for forming an inlet opening (22),
- closing the main mould jowls (8) for forming the hollow mould interior for the dispensing container (1), and simultaneous welding of the plastic hose (6) in its front area for forming the container floor,
- expanding of the plastic hose (6) for abutment of the same against the mould walls of the main mould jowls (8) by blowing air through the inlet opening (22) and/or by creating a vacuum on the mould walls,
- filling of the container (1) located within the mould with the medium that is to be dispensed through the inlet opening (22),
- insertion of said closure unit (3) through the section of the plastic hose (6) extending through the header jowls (10), which comprises a dispensing needle (11) for the medium projecting from its outermost end, the same comprising a safety means (5, 17), the first component (17) of which comprises a needle protection body (19) that can be displaced along the needle (11) between a projected protected position and a retracted usage position, and
- closing of the header jowls (10), and therefore moulding of the section of the plastic hose (6) extending through the header jowls (10) into a safety cover (5) which encloses the needle (11) of the closure unit (3) as a second component of the safety means (5, 17).

2. Moulding method according to Claim 1, **characterised in that** the steps of opening out and filling the container (1) within the mould are carried out simultaneously by means of a combined blowing/filling spike (24) projecting through the inlet opening (22).

3. Moulding method according to Claim 1 or 2, **characterised in that** a pre-formed breaking point (7) is formed in the wall of the same in the area of the transition between closure unit (3) and safety cover (5) when the header jowls (10) of the mould are closed.

4. Moulding method according to Claim 3, **characterised in that** at least one projecting twist-off knob (9) is formed on the safety cover (5) as a turnable knob for separating the safety cover (5) at the pre-formed breaking point (7) when the header jowls (10) are closed.

5. Moulding method according to Claim 3 or 4, **characterised in that** the closure unit (3) is inserted into a seat (27) with its closure body (4), the same being formed by the mould wall sections of the closed main mould jowls (8) adjacent to the header jowls (10).

6. Moulding method according to Claim 5, **characterised in that** a closure unit (3) is inserted, the closure body (4) of which extends through the seat (27) formed by the main mould jowls (8) along the needle (11) into the area of the header jowls (10).

7. Moulding method according to Claim 6, **characterised in that** the pre-formed breaking point (7) is formed at a distance from the seat (27) of the closure body (4) in such a way that it encloses the closure body (4) within the area projecting into the header jowls (10).

## Revendications

1. Procédé de moulage, en particulier procédé de moulage par soufflage et/ou sous vide, pour la fabrication d'un récipient (1) rempli d'un milieu à distribuer, qui présente les étapes :
- introduction d'un tuyau en matière plastique (6) extrudé dans un moule ouvert qui présente des coquilles de moulage principales (8) mobiles et des coquilles de tête (10),
- sectionnement du tuyau en matière plastique (6) dans la zone située à l'extérieur des coquilles de tête (10) pour la formation d'une ouverture d'introduction (22),
- fermeture des coquilles de moulage principales (8) pour la formation de l'empreinte de moule pour le récipient distributeur (1), et la réalisation à cette occasion du soudage du tuyau en matière plastique (6) sur sa zone avant pour la formation du fond du récipient,
- élargissement du tuyau en matière plastique (6) pour l'appui sur les parois de moule des coquilles de moulage principales (8) par l'insufflation d'air par l'ouverture d'introduction (22) et/ou par la production d'un vide sur les parois de moule,
- remplissage du récipient (1) se trouvant dans le moule avec le milieu à distribuer, par l'ouverture d'introduction (22),
- pose, dans le tronçon du tuyau en matière plastique (6) passant à travers les coquilles de tête (10), d'une unité de scellement (3) qui possède une canule de distribution (11) du milieu dépassant de son extrémité extérieure, et qui a un équipement de blocage (5, 17), dont le premier composant (17) possède un corps de protection de canule (19) qui peut coulisser sur la canule (11) entre une position de protection poussée vers l'avant et une position d'utilisation tirée vers l'arrière, et
- fermeture des coquilles de tête (10) et, de ce fait, moulage du tronçon du tuyau en matière plastique (6) passant à travers les coquilles de tête (10) pour former un capot de protection (5), qui entoure la canule (11) de l'unité de scellement (3) en tant que deuxième composant de l'équipement de blocage (5, 17).

2. Procédé de moulage selon la revendication 1, **caractérisé en ce que** les étapes d'élargissement et de remplissage du récipient (1) situé dans le moule sont exécutées en commun à l'aide d'un mandrin de soufflage/remplissage (24) combiné pénétrant dans l'ouverture d'introduction (22).

3. Procédé de moulage selon la revendication 1 ou 2, **caractérisé en ce que**, lors de la fermeture des coquilles de tête (10) du moule, il est formé, dans la zone de transition entre l'unité de scellement (3) et le capot de protection (5), sur sa paroi, un emplacement destiné à la rupture (7).

4. Procédé de moulage selon la revendication 3, **caractérisé en ce que**, lors de la fermeture des coquilles de tête (10) au niveau du capot de protection (5), il est formé au moins une poignée (9) en saillie en tant que manette de torsion pour séparer le capot de protection (5) au niveau de l'emplacement destiné à la rupture (7).

5. Procédé de moulage selon la revendication 3 ou 4, **caractérisé en ce que** l'unité de scellement (3) avec son corps de scellement (4) est posée dans un siège (27) qui est formé par les tronçons de paroi de moule, voisins des coquilles de tête (10), des coquilles de moulage principales (8) fermées.

6. Procédé de moulage selon la revendication 5, **caractérisé en ce qu'**il est mis une unité de scellement (3), dont le corps de scellement (4), s'étend le long de la canule (11), du siège (27) formé par les coquilles de moulage principales (8) à la zone des coquilles de tête (10).

7. Procédé de moulage selon la revendication 6, **caractérisé en ce que** l'emplacement destiné à la rupture (7) est constitué à distance du siège (27) du corps de scellement (4) de sorte qu'il entoure le corps de scellement (4) dans la zone faisant saillie dans les coquilles de tête (10).
